# EUROPEAN PATENT APPLICATION

(11) **EP 4 706 789 A1**
(43) Date of publication of application: **11.03.2026**
(21) Application number: 24823004.7
(22) Date of filing: 10.01.2024
(51) Int. Cl.: A63B 24/00, A61H 1/02, A63F 13/53, G16H 20/00

(54) **INFORMATION PROCESSING SYSTEM, INFORMATION PROCESSING METHOD, AND INFORMATION PROCESSING PROGRAM**

(30) Priority: 13.06.2023 JP 2023097169
(71) Applicant: mediVR, Inc., Toyonaka City, Osaka 561-0872 (JP)
(72) Inventor: HARA Masahiko, Toyonaka City, Osaka 5610872 (JP)
(74) Representative: Betten & Resch
(86) International application number: PCT/JP2024/000301
(87) International publication number: WO 2024/257380

(57) **Abstract**

This invention effectively improves the cognitive ability or the exercise ability of a user. An information processing system includes a blocker that makes whole or a part of a body of a user invisible, a requester that requests the user to perform a body action that makes a part of the body of the user or an object operated by the user overlap a target object, and a controller that controls, under an environment which is generated by the blocker and the requester and in which an abnormal action of a joint is visualized, difficulty of the body action in accordance with a motion of a joint part different from a body that performs the body action.

## Description

### TECHNICAL FIELD

The present invention relates to an information processing system, an information processing method, and an information processing program.

### BACKGROUND ART

In the above technical field, patent literature 1 discloses a system that is performed for a patient suffering hemiplegia from a stroke.

### CITATION LIST

### PATENT LITERATURE

Patent literature 1: Japanese Patent Laid-Open No. 2015-228957

### NON-PATENT LITERATURE

Non-Patent literature 1: Nature "A somato-cognitive action network alternates with effector regions in motor cortex" by Evan M. Gordon et al. Published online: 19 04 2023

### SUMMARY OF THE INVENTION

### TECHNICAL PROBLEM

However, in the technique described in the above literature, there is no index indicating whether to advance to the next step when a user makes what kind of action or when he/she does not make what kind of action. On the other hand, as disclosed in non-patent literature 1, concerning a neural circuit in the brain, it has become known that there exists a cranial nerve region that controls a coordination movement in which a plurality of joints link.

The present invention enables to provide a technique of solving the above-described problem.

### SOLUTION TO PROBLEM

One example aspect of the present invention provides an information processing system comprising:
a blocker that makes whole or a part of a body of a user invisible;
a requester that requests the user to perform a body action that makes a part of the body of the user or an object operated by the user overlap a target object; and
a controller that controls, under an environment which is generated by the blocker and the requester and in which an abnormal action of a joint is visualized, difficulty of the body action in accordance with a motion of a joint part different from a body that performs the body action.

Other example aspect of the present invention provides an information processing method comprising:
making, by a blocker, whole or a part of a body of a user invisible;
requesting, by a requester, the user to perform a body action that makes a part of the body of the user or an object operated by the user overlap a target object; and
controlling, by a controller, under an environment which is generated in the making and the requesting and in which an abnormal action of a joint is visualized, difficulty of the body action in accordance with a phenomenon called an articular linkage in which a motion of a joint part different from a body that performs the body action.

Still other example aspect of the present invention provides an information processing program for causing a computer to execute a method, comprising:
making whole or a part of a body of a user invisible;
requesting the user to perform a body action that makes a part of the body of the user or an object operated by the user overlap a target object; and
controlling, under an environment which is generated in the making and the requesting and in which an abnormal action of a joint is visualized, difficulty of the body action in accordance with a motion of a joint part different from a body that performs the body action.

### ADVANTAGEOUS EFFECTS OF INVENTION

According to the present invention, it is possible to effectively improve the cognitive ability or the exercise ability of a user.

### BRIEF DESCRIPTION OF DRAWINGS

Fig. 1 is a block diagram showing the configuration of an information processing system according to the first example embodiment;
Fig. 2A is a block diagram showing the configuration of a rehabilitation support system according to the second example embodiment;
Fig. 2B is a view for explaining the configuration of the rehabilitation support system according to the second example embodiment;
Fig. 2C is a view for explaining the configuration of the rehabilitation support system according to the second example embodiment;
Fig. 3A is a view showing an example of the operation panel screen of the rehabilitation support system according to the second example embodiment;
Fig. 3B is a view showing another example of the operation panel screen of the rehabilitation support system according to the second example embodiment;
Fig. 4A is a view showing an example of the task data table of the rehabilitation support system according to the second example embodiment;
Fig. 4B is a view showing another example of the task data table of the rehabilitation support system according to the second example embodiment;
Fig. 5 is a view showing an example of a display screen on the head mounted display of the rehabilitation support system according to the second example embodiment;
Fig. 6 is a view showing an example of a display screen on the head mounted display of the rehabilitation support system according to the second example embodiment;
Fig. 7 is a view showing an example of a display screen on the head mounted display of the rehabilitation support system according to the second example embodiment;
Fig. 8 is a view showing an example of a display screen on the head mounted display of the rehabilitation support system according to the second example embodiment;
Fig. 9 is a flowchart showing the procedure of processing of the rehabilitation support system according to the second example embodiment; and
Fig. 10 shows data showing a result of rehabilitation by the rehabilitation support system according to the second example embodiment.

### DESCRIPTION OF EXAMPLE EMBODIMENTS

Example embodiments of the present invention will now be described in detail with reference to the drawings. It should be noted that the relative arrangement of the components, the numerical expressions and numerical values set forth in these example embodiments do not limit the scope of the present invention unless it is specifically stated otherwise.

### [First Example Embodiment]

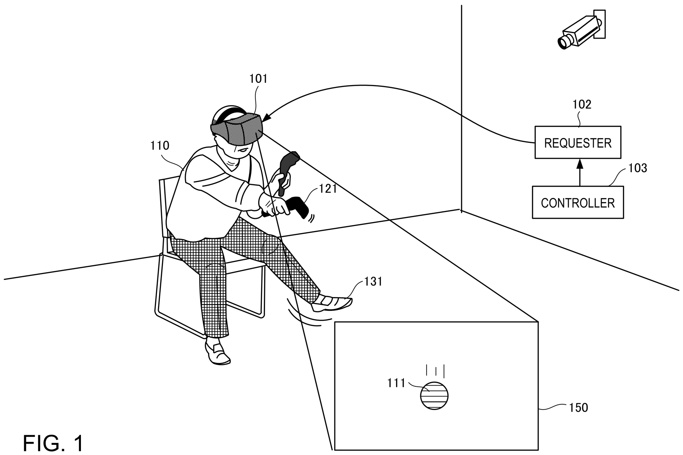

An information processing system 100 according to the first example embodiment of the present invention will be described with reference to Fig. 1. The information processing system 100 is a system that provides an environment to visualize an abnormal action of a joint.

As shown in Fig. 1, the information processing system 100 includes a blocker 101, a requester 102, and a controller 103. The blocker 101 makes the whole or a part of the body of a user 110 invisible. The requester 102 requests the user 110 to perform a body action that makes a part of the body of the user 110 or an object 121 operated by the user overlap a target object 111. The controller 103 controls the difficulty of the body action in accordance with a motion 131 of a joint different from a body part the user 110 moves to make the body action.

With the above-described configuration, it is possible to visualize an abnormal action of the joint different from the body part the user moves to make the body action, and in turn, it is possible to effectively improve the cognitive ability or the exercise ability of the user using the abnormal action as an index.

### [Second Example Embodiment]

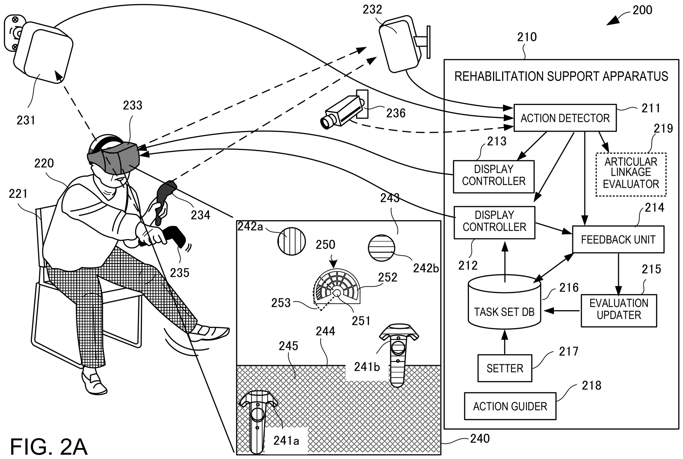

A rehabilitation support system 200 according to the second example embodiment of the present invention will be described with reference to Fig. 2A. The rehabilitation support system 200 is an example of a system that provides an environment to visualize an abnormal action of a joint, and the present invention is not limited to this. That is, the present invention is not limited to the concept "rehabilitation", and physical illnesses from paralysis and disorder to dementia and schizophrenia and treatment systems that actively treat cognitive and mental disorders are also incorporated in the present invention. Furthermore, action verification systems and ability improving systems configured to improve the exercise ability or cognitive ability of nonhandicapped people who do not need a particular treatment or rehabilitation as compared to current situations are also incorporated in the present invention.

Fig. 2A is a block diagram for explaining the configuration of the rehabilitation support system 200 according to this example embodiment. The rehabilitation support system 200 according to this example embodiment improves at least one selected from a group of bodily functions such as the upper limb function, the walking function, the trunk function, and the balance function, cognitive functions (including the spatial cognitive function, the attention function, and the higher brain function), and sensory functions (including the inner ear, the vestibular system, the tactile sensation, the temperaesthesia and pain sensation, the position sensation, and the depth sensation).

As shown in Fig. 2A, the rehabilitation support system 200 includes a rehabilitation support apparatus 210 serving as a requester and a controller, two base stations 231 and 232, a head mounted display 233 serving as a blocker, and two controllers 234 and 235. Sitting on a chair 221, a user 220 wears the head mounted display 233, and moves the body in accordance with display on the head mounted display 233. In this example embodiment, a description will be made assuming verifying an action performed while sitting on a chair. However, the present invention is not limited to this, and the action may be made while standing or walking, or on a bed, at a supine position or a prone position, or while running or performing another specific action. Also, the user 220 holds the controllers 234 and 235 by both hands here, but the present invention is not limited to this, and the controllers may be held on or attached to body parts other than hands, such as feet or trunk.

The two base stations 231 and 232 sense the motion of the head mounted display 233 and the motions of the controllers 234 and 235, and send these to the rehabilitation support apparatus 210. The rehabilitation support apparatus 210 controls display on the head mounted display 233 based on the motion of the head mounted display 233. More specifically, the position and direction of a viewpoint on a virtual space are changed in accordance with the position and direction of the head mounted display 233, and an image to be displayed on the head mounted display 233 is decided. The rehabilitation support apparatus 210 also evaluates the rehabilitation action of the user 220 based on the motions of the controllers 234 and 235.

Note that the head mounted display 233 can be of a non-transmissive type, a video see-through type, an optical see-through type, or a spectacle type. In this example embodiment, a virtual space of VR (Virtual Reality) is presented to the user. However, a real space and a virtual space may be displayed in a superimposed manner, like AR (Augmented Reality), physical information may be reflected on a virtual space, like MR (Mixed Reality), or a hologram technology may be used as an alternative means.

Here, it is important to make the whole or a part of the body of the user directly invisible by the head mounted display 233. In the real space, normally, a human reaches a target while visually recognizing the motion of his/her body and unconsciously finely adjusting the motion in the brain. In this system, since the user 220 cannot directly view the hands of his/her own in the real space, the brain of the user cannot accurately correct the motions of the hands as usual. For this reason, a coordination movement disorder is visualized, and an abnormal action of a joint different from a body part that makes the body action can readily be detected. At this time, for example, as the blocker, a projection mapping system that displays background on the whole or a part of the body of the user may be employed. Also, as the blocker, a contact lens type display that displays a virtual space may be employed.

In this example embodiment, as an example of a sensor configured to detect the position or action of the hand or head of the user, the controllers 234 and 235 held in the hands of the user 220 and the base stations 231 and 232 have been described. However, the present invention is not limited to this. A camera (including a depth sensor) configured to detect the actions or "the positions of the hands themselves" of the user by image recognition processing, or a sensor configured to detect the positions of the hands of the user by a temperature, a wristwatch-type wearable terminal put on an arm of the user, a motion capture device, or the like can also be applied to the present invention interlockingly with an action detector 211. That is, using a three-dimensional tracking device such as Kinect^{®} or an action analysis device or attaching a marker to the body is also one example embodiment.

The rehabilitation support apparatus 210 includes the action detector 211, display controllers 212 and 213, a feedback unit 214, an evaluation updater 215, a task set database 216, a setter 217, and an action guider 218.

The action detector 211 acquires, via the base stations 231 and 232, the positions of the controllers 234 and 235 held in the hands of the user 220, and detects the rehabilitation action of the user 220 based on changes in the positions of the hands of the user 220.

The display controller 212 generates and displays, in a virtual space 240, target objects 242a and 242b configured to urge the user 220 to do a three-dimensional body action. In particular, the display controller 212 generates, in the virtual space 240, the target object 242a configured to cause a part of the left-side body of the user 220 to perform a rehabilitation action and the target object 242b configured to cause a part of the right-side body of the user 220 to perform a three-dimensional rehabilitation action. That is, the display controller 212 functions as a requester that requests the user to do reaching to the target object.

Note that, here, the display controller 212 requests reaching to a virtual target displayed on the head mounted display, but the present invention is not limited to this, and the target may be an object provided in the real space. The target may be an object suspended by two or more wires or an object floating in the air by an ultrasonic vibration. Alternatively, the target may be an object that is three-dimensionally displayed by hologram. The shape of the target object that is the target is not limited to the spherical shape and may be a triangular shape, a rectangular shape, or a dish shape. It may be the shape of a certain character. The concept of the present invention also includes a system that allows the user to visually recognize only the target while making the motion of the user himself/herself invisible by partially blocking the vision of the user using an optical see-through type or a smartglass type head mounted display.

The display controller 212 generates the target objects 242a and 242b in the virtual space and moves these downward from above the user 220. Accordingly, on the head mounted display 233, each target object is displayed such that the display position and size gradually change (for example, gradually becomes large and then becomes small). Note that as for the moving direction of the target object, for example, the target object may rise from the floor surface to above the head. Also, including a movement in the depth direction or the left-and-right direction in addition to the movement in the vertical direction, the target object may make any three-dimensional movement, or the target object may be fixed at a specific coordinate position without moving depending on the stage of the user.

Furthermore, the display controller 212 generates, in the virtual space, avatar objects 241a and 241b that move in accordance with a detected user action. Here, the avatar object 241a is an object that moves in correspondence with the controller 234 operated by the left hand and indicates the position of a part of the left-side body of the user. The avatar object 241b is an object that moves in correspondence with the controller 235 operated by the right hand and indicates the position of a part of the right-side body of the user. The avatar object is not limited to an object that moves in correspondence with the position of the controller. It may be a virtual object that moves along with the change of the position of a part of the body of the user using the three-dimensional body tracking technique, as described above. The object operated by the user is not limited to the controller as shown in Fig. 2A and may be, for example, an object like a bat or a golf club. The display controller 212 requests the user to perform such a body action that makes the avatar objects 241a and 241b overlap the target objects 242a and 242b.

In a case where a part or whole of the body is invisibly blocked, the burden in the brain of the user is large as compared to a case where the actual body motion is directly visible, even if the avatar objects 241a and 241b are displayed. This is because there is necessity of a brain work (so-called point estimation) for estimating the position (point) of the controller in the real space by viewing the position (point) of the controller in the virtual space in a state in which the body of the user (including the controller in the real space) is invisible. In this case, the brain needs to quite strongly image the state of the body without resort to vision, and this is expressed in medicine as "strong feed forward is needed". For the brain that is requested to do the strong feed forward, it is more difficult to coordinately adjust the body. Hence, a coordination movement disorder is visualized, and an abnormal motion of a joint different from a part that performs the body action readily appears. Also, when the strong feed forward requiring point estimation is performed, contraction of a deep muscle in the body can be caused by a stimulus via the pyramidal tract.

For this reason, for example, when the user moves the right arm to make the avatar object 241b of the right controller 235 reach the target object 242b on the right side, he/she may unconsciously apply a force to the knee joint of the left leg, and the lower thigh part of the left leg may move to the front or rear side. Here, the phenomenon that the joint that is unnecessary for the requested motion unintentionally moves is called an articular linkage and is used as an index to improve the action of the user. Note that the articular linkage is visualized even without an anatomical linkage called an anatomical train. For example, when voluntarily moving the joint of a finger of a foot, an abnormality occurs in the jaw joint, the hip joint, or the shoulder joint.

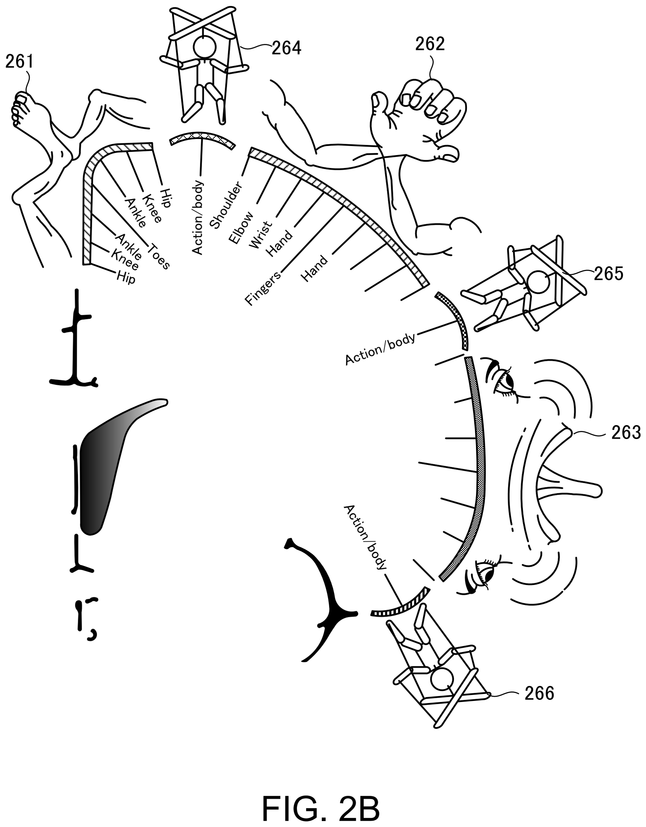

It can be considered that this phenomenon is supported by the fact that the motor areas that control a coordination movement exist across all joints (Fig. 2B), as is mentioned in non-patent literature 1. Referring to Fig. 2B, in the latest neurology, it is found that cranial nerves are put into groups for symmetrical parts (for example, the right and left hands, the right and left legs, and the right and left eyes), and the motor areas thereof are arranged symmetrically in the brain. It has also recently been known that it seems that neural regions that control coordination of body parts, represented by puppets 264 to 266, exist between a motor area 261 of legs and a motor area 262 of hands and between the motor area 262 of hands and a motor area 263 of face. It is considered that because of the existence of the nerves that make the body parts coordinate, like the puppets 264 to 266, the articular linkage during a movement is visualized under a specific environment according to the present invention in which point estimation is required in a situation where the whole or a part of the body is invisible.

In fact, the present inventor created the present invention by observing actual rehabilitation actions of users before publication of non-patent literature 2, but it can be said that the usefulness and logic are theorized by the paper of non-patent literature 1.

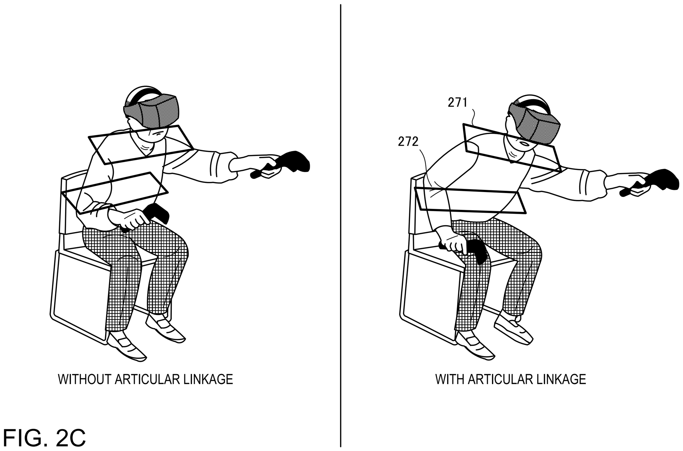

Fig. 2A shows the motion of a knee joint as an example of appearance of the articular linkage. However, the present invention is not limited to this, and all involuntary movements of joints different from the body part that performs the requested action are included in the concept of the present invention. For example, as shown in Fig. 2C, in some cases, joint planes 271 and 272 are sensed, and the tilts thereof are calculated, thereby detecting simultaneous occurrence of a twist of a shoulder joint or trunk (a cervical vertebral joint or thoracic vertebral joint) and a twist of a hip (a lumbar vertebral joint or a hip joint).

More specifically, a twist of a cervical intervertebral joint (this appears in the direction of the head), a twist of a thoracic intervertebral joint (this appears in the angle of the shoulder), elevation or abduction of a shoulder joint (the armpit is open), extension/contraction of a knee joint, a hip joint, or a leg joint (a knee or toe moves up/down or is inverted or abducted), extension/contraction of an elbow joint (an arm that is not requested to be moved is fixed at a flexion position), extension/contraction of a jaw joint (the mouth is open), flexion/extension of a joint in hand or a finger joint, and flexion of a toe joint (claw toe) or extension occur as articular linkages. Such motions of joints include not only flexion and extension but also adduction, abduction, internal rotation, external rotation, circumduction, combinations thereof, and motions that are not mentioned here.

A camera 236 may be installed, and an articular linkage evaluator 219 that detects and evaluates the position, twist, tilt, motion speed, and acceleration of each joint of the user during a rehabilitation action may be provided. The articular linkage evaluator 219 detects the motion of each joint of the user 220 and evaluates, based on the magnitude, speed, and timing of the motion, whether an articular linkage occurs. A rehabilitation action is requested such that the articular linkage becomes small, and the user is caused to achieve it. Comparing the brain to a CPU with a calculation ability of 100, if the information processing process for an articular linkage, which needs 80 of the ability, is reorganized to 20, an action that is more complex than the request can be performed, and an exercise ability can be improved. Alternatively, if the CPU capacity usable for cognitive processing improves, the cognitive ability is also improved. When the user is shot by the camera 236, and the shot image is compared with an image in a normal state (before the motion) or compared with a normal action model, an abnormal twist or motion of the joint plane can be detected. Alternatively, sensors are adhered to joints (for example, both shoulders, both elbows, wrists, both knees, and both leg joints) of the user (or motion capture is used), and the positions of the joints are detected by the base stations 231 and 232, thereby sensing an articular linkage. An operator may visually recognize an occurrence of an articular linkage and input it to the system, as a matter of course. The tilt of the head mounted display 233 may be detected by the base stations 231 and 232 and the articular linkage of the neck may be sensed. For example, if the head mounted display 233 tilts by 30°, it can be judged that the angle of the trunk is 15° (determination based on a camera image) and the angle of the neck is 15°. The tilt of a wrist joint or the like can be determined by detecting the positions and tilts of the controllers 234 and 235 by the base stations 231 and 232. Furthermore, the articular linkage evaluator 219 may express a motion of an articular linkage as a distance or an acceleration using heat map on a bull's eye, or may express a time-rate change of an articular linkage and provide information useful for action difficulty control. More specifically, how the articular linkage changes from the start to the end of a requested reaching action may be visualized, or the magnitudes of the articular linkages of joints may be compared. This facilitates judging which joint should be focused.

Six joints including the hip joint, the knee joint, the leg joint, the neck joint, the trunk, and the elbow joint may intensively be sensed. The joints may be divided into more classifications, and all joint units (68 joints for a human) may be observed, or reversely, a contrivance may be made to place focus on only one or a small number of joints for which an abnormality is readily sensed. Also, a plurality of evaluation indices may be put into one integrated index or parameter as a total point. That is, the articular linkages of the whole body or important parts may be weighted and integrated, and total articular linkage evaluation (calculation of an articular linkage score) may be performed.

In a requested action of overlaying a sensor of 2 cm on a target of 10 cm, the accuracy of point estimation is low. In a requested action of overlaying a sensor of 1 cm on a target of 1 cm, accurate point estimation is necessary. In this case, if a blocker such as a head mounted display is used, the user is in a handicapped state because visual information in the real world is blocked. There is necessity of activating the cranial nerve for point estimation, and a coordination movement disorder is visualized, that is, an articular linkage more conspicuously appears. This is one of articular linkage generation mechanisms.

If the rehabilitation level (the exercise ability of the user) rises, the required accuracy of point estimation is raised by making the sizes of both the target and the sensor small, and an articular linkage that is a compensation is more strongly visualized. When the rehabilitation action is repetitively requested in that state or a guidance is made by call-out or physical contact such that the user can more easily perform a correct action, the articular linkage subsides. This can achieve a rewired state with alignment of the whole body and improve the coordination movement disorder.

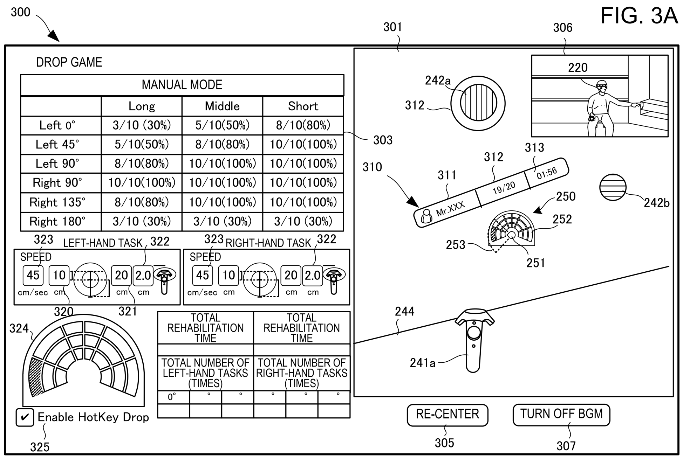

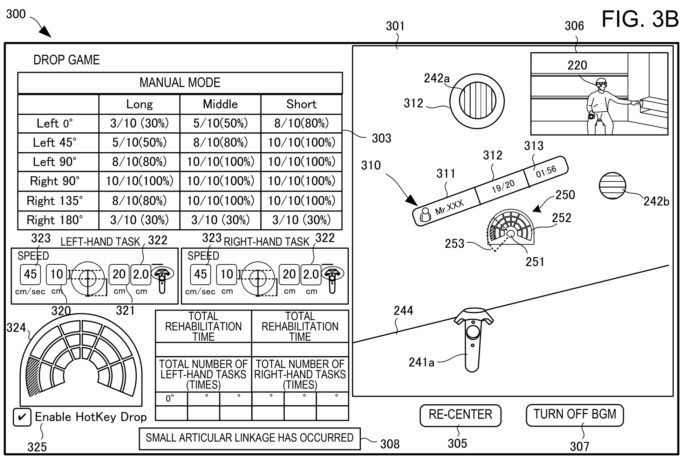

The articular linkage evaluator 219 may display the position and size of a sensed articular linkage on an operator screen (Fig. 3A and 3B) or the like. A comparator that performs comparison with a past articular linkage or comparison with a normal action model may further be provided, and the operator may be notified of the comparison result. This allows the operator to more accurately recognize a change of the articular linkage.

If it is determined that the articular linkage has decreased to a certain reference or less, the load of the rehabilitation action is increased. If it is determined that the articular linkage has increased to the certain reference or more or the large state continues (the articular linkage is not improved), the load of the rehabilitation action is decreased.

Note that concerning whether an articular linkage appears, the size, angle, height, distance, and motion direction of a target object, the presence/absence of a motion, the motion speed, color, shape, and visibility, the size, color, shape, and visibility of an index or a device such as a controller for overlap in point estimation, the presence/absence of background information, and the presence/absence of background sound have influence. An object of the rehabilitation action in this system is to make an articular linkage appear and eliminate this. That is, the articular linkage is the guideline for the treatment. The articular linkage can sometimes be eliminated by pushing the knee or shoulder of the user during the rehabilitation action. That is, the disorder of the neural circuit in the brain can be forcibly corrected by hand.

Referring back to Fig. 2A, the images of the avatar objects 241a and 241b and the target objects 242a and 242b are displayed on the display screen 240 in accordance with the direction and position of the head mounted display 233 detected by the action detector 211. The images of the avatar objects 241a and 241b and the target objects 242a and 242b are superimposed and displayed on a background image 243. Here, the avatar objects 241a and 241b have the same shape as the controllers 234 and 235. However, the present invention is not limited to this, and these may have a hand shape. In addition, the size, shape, and color may be changed between the left and right sides. One or more buttons are prepared for each of the controllers 234 and 235, and the controllers are configured to do various kinds of settings including initial setting such as origin setting by operating the buttons. However, the button function may be disabled, or instead of arranging the buttons, all settings may be executed using an external operator. The background image 243 is cut out from the virtual space including a horizon 244 and a ground surface object 245. How the background image 243, the avatar objects 241a and 241b, and the target objects 242a and 242b are seen changes depending on the movement of the sight line (the position and direction of the head mounted display).

The display controller 212 generates an avatar object indicating the position of a part of the right-side body and a target object indicating the target position of the avatar object on the right side in similar colors or in the same shape. Also, the display controller 212 generates an avatar object indicating the position of a part of the left-side body and a target object indicating the target position of the avatar object on the left side in similar colors or in the same shape. The colors or shapes of the left-side avatar object and the left target object are preferably set such that these are different from the colors or shapes of the right-side avatar object and the right target object. However, the avatar object and the target object on the same side need not always be set to similar colors or the same shape, and the color need not necessarily be changed between the right side and the left side. These settings can be done any way in accordance with the user's feeling of use.

Here, as an example, the avatar objects 241a and 241b are colored in, for example, blue and red, and the target objects 242a and 242b are also colored in blue and red. The blue avatar object 241a is brought into contact with the blue target object 242a, thereby achieving a task. Similarly, the red avatar object 241b is brought into contact with the red target object 242b, thereby achieving a task. That is, even if the avatar object of the different color is brought into contact, the task cannot be achieved. An example in which the objects are colored in blue and red is shown here. However, the display may be done in another coloring (yellow and green or the like) for a blind user. Alternatively, the objects may be discriminated by using not colors but shapes, using a linguistic notation such as "left", "right", "L (Left)", or "R (Right)", expressing a symbol such as a star, a triangle, or O?, or expressing different patterns (stripes) like the target objects 242a and 242b in Fig. 2A.

The action guider 218 provides guidance such that the rehabilitation action of a part of the right-side body of the user and the rehabilitation action of a part of the left-side body of the user are alternately performed. More specifically, the action guider 218 guides the rehabilitation action of the user by displaying characters, colors, or graphics representing right and left, outputting a voice for identifying "right" and "left", or giving tactile stimulation using the vibration of the controllers the user holds in the left and right hands, or by using a method of touching or patting a shoulder or an arm.

When the avatar objects 241a and 241b hit the target objects 242a and 242b, the display controller 212 makes the target objects 242a and 242b disappear. Determining that the target action is achieved, the feedback unit 214 performs visual feedback by displaying a message for the purpose of notifying or informing the achievement of the target action. If the sensor centers of the avatar objects 241a and 241b reach the centers of the target objects 242a and 242b, "excellent" is displayed. Even if reaching the peripheral region, "well done" is displayed for every task. Thus, the achievement of each requested body action is fed back to the user. The feedback unit 214 need only perform the feedback by stimulating one or more sensations of the five senses of the user. If the feedback is performed by stimulating two or more sensations of the five senses of the user, the cognitive ability or the exercise ability of the user or both of them can be improved more effectively.

That is, the feedback unit 214 notifies achievement of each rehabilitation action of the user 220 for the target objects 242a and 242b. Various methods can be used as the notification method. The user may be made to know the degree of achievement of the target action by temporarily displaying characters "excellent" or "well done" in the display screen, or the user may be informed of the degree of achievement of the target action using a similar voice or sound effect. Furthermore, of the controllers 234 and 235, only the controller that has made the action to contact the target object may be vibrated to notify the user of the degree of achievement of the target action. Alternatively, as for the notification of achievement, for example, the degree of achievement may be notified using stepwise classifications such as "perfect achievement", "imperfect achievement", and "no achievement".

For example, if the shortest distance between a sensor object included in the avatar object 241a or 241b and the target object 242a or 242b falls within a predetermined range, the target is achieved, and the target object 242a or 242b disappears. At this time, if the shortest distance between the target object 242a or 242b and the sensor object (for example, a spherical object including the center point of the tip of the avatar object 241a or 241b) included in the avatar object 241a or 241b is equal to or less than a threshold, the target is completely achieved. For example, "excellent" is displayed, and a corresponding voice is output simultaneously to make feedback. The feedback that stimulates a plurality of different sensations is called multi-channel biofeedback. At the same time, the controllers 234 and 235 may be vibrated, or stimuli may be imparted to the sense of smell or the sense of taste. That is, as the feedback, the feedback unit 214 can stimulate one sensation of the five senses, that is, the sense of sight, the sense of hearing, the sense of touch, the sense of taste, and the sense of smell. For the sensation stimulus, two sensations may be combined, three or more sensations may be combined, or all sensations may be stimulated. In a situation where point estimation is performed, if feedback is performed every time a requested action is achieved, a trigger to cause the brain to correct and improve the coordination movement disorder can be obtained. That is, and articular linkage and a coordination movement disorder are improved. As the number of sensations stimulated in feedback increases, the improvement efficiently progresses or the improvement is retained for a long time. For example, as compared to feedback of one sensation, if feedback of two sensations is performed, a symptom to be improved in several days is improved by the hour. Alternatively, as compared to feedback of two sensations, if feedback of three sensations is performed, a symptom to be improved in several hours is improved by the minute. Furthermore, even for a symptom that is improved by feedback of one sensation but returns immediately, when feedback of two sensations is performed, the effect of improvement can be maintained for several days. Alternatively, even for a symptom that is improved by feedback of two sensations but returns immediately, when feedback of three sensations is performed, the effect of improvement can be maintained for several weeks. To express the characteristic of the treatment, in a medical scene, the treatment is also expressed as "brain reprogramming therapy (BRT)", "brain re-wiring therapy (BRT)", or "motor coordination therapy (MCT)".

Depending on the degree of decrease of the distance between the sensor object and the target objects 242a and 242b, a rehabilitation action may be evaluated in three or more levels. Also, two or more target objects may simultaneously be generated in the virtual space and displayed.

The display controller 213 displays a radar screen image 250 on the display screen 240 of the head mounted display 233. The radar screen image 250 is a notification image used to notify the user of generation of a target object 152. The radar screen image 250 notifies the user of the direction in which the positions of the generated target objects 242a and 242b are located relatively with respect to a reference direction (initially set in the front direction of the user who is sitting straight on a chair) in the virtual space. The radar screen image 250 also notifies the user how far the positions of the generated target objects 242a and 242b are apart from the user 220. Note that the notification image is not limited to the radar screen image, and the notification may be made using characters, an arrow, a symbol, an illustration, or a type, intensity, blinking, or the like of light or a color. The notification method is not limited to the image, and may use a voice, a vibration, or a combination of some of a voice, a vibration, and an image. Independently of the direction of the head of the user 220, the display controller 213 displays the radar screen image 250 at the center (for example, within the range of -50° to 50°) of the display screen 240 of the head mounted display 233. However, the display portion is not limited to the center, and may be, for example, an arbitrary place on the four corners, the upper end, the lower end, the left end, and the right end of the screen. Based on the information of positions, angles, and number of target objects displayed on the radar screen, the patient can estimate the difficulty of an exercise action that should be performed next. If a more difficult action is predicted depending on the patient, an articular linkage is more visualized. That is, it can be said that the display controller functions as a controller configured to control the difficulty of an action requested of the patient.

The radar screen image 250 includes a head image 251 representing the head of the user viewed from above, a block image 252 obtained by dividing the periphery of the head image 251 into a plurality of blocks, and a fan-shaped image 253 as a visual field image representing the visual field of the user. A target position image representing the position of a target object is shown by coloring, blinking, or lighting a block in the block image 252. This allows the user 220 to know whether the target object exists on the left side or the right side with respect to the direction in which he/she faces. Note that in this example embodiment, the block image 252 is fixed, and the fan-shaped image 253 moves. However, the present invention is not limited to this, and the block image 252 may be moved in accordance with the direction of the head while fixing the fan-shaped image 253 and the head image 251. More specifically, if the head turns to the left, the block image 252 may rotate to right.

The feedback unit 214 preferably changes the message type via the display controller 212 in accordance with evaluation of the rehabilitation action. For example, if the sensor object contacts the center of the target object 242a or 242b, "excellent" is displayed. If the sensor object contacts only the peripheral portion of the center of the target object 242a or 242b, "well done" is displayed. The sizes of the target objects 242a and 242b and the size of the peripheral portion can be set by the setter 217. The size of the sensor object can also be set by the setter 217. If the shortest distance between the target object 242a or 242b and the sensor object included in the avatar object 241a or 241b is not more than a first threshold, "excellent" is displayed because of the perfect achievement of the target, and a corresponding voice "excellent" is output to make feedback. If the shortest distance between the target object 242a or 242b and the sensor object included in the avatar object 241a or 241b is not less than the first threshold and not more than a second threshold, "well done" is displayed because the target is achieved, and a corresponding voice "well done" is output to make feedback. Note that the output voice need not be the same as the message, and a nonverbal sound effect, for example, "dingdong" may be used. Note that since a more accurate action is requested as the distance necessary for the contact between the sensor object and the target object becomes short, the brain needs to request a more accurate and advanced action from the body as a motion instruction. As described above, image giving to the brain is called feed forward. That is, the shorter the distance between the sensor object and the target object required for completion of an action is, the stronger the required feed forward is. This can continuously adjust the load level of the motion, cognition, and sensation of the user.

The feedback unit 214 performs feedback to impart stimuli to two or more sensations of, for example, the five senses (sense of sight, sense of hearing, sense of touch, sense of taste, and sense of smell) of the user who has virtually touched the target objects 242a and 242b almost at the same time as the timing at which the sensor object enters a predetermined distance from the center of the target object 242a or 242b or the timing at which the sensor object contacts the target object 242a or 242b (called real-time multi-channel biofeedback or immediate multi-channel biofeedback). The effect is large if a delay from the timing to feedback is, for example, 1 sec or less. The shorter the interval between the action timing of the user and the timing of feedback is (the smaller the delay is), the larger the effect is. While performing feedback of giving stimuli to the sense of sight of the user by an image "excellent!", the feedback unit 214 simultaneously performs feedback of giving stimuli to the sense of hearing of the user by a voice or sound effect output from a speaker. The notification of task achievement to five-sense stimulus can be done by any combination, for example, in accordance with the action type.

Furthermore, the feedback unit 214 may simultaneously output feedback of giving stimuli to the sense of sight of the user 220 by the image "excellent!", feedback of giving stimuli to the sense of hearing of the user 220 by the voice output from the speaker, and feedback of giving stimuli to the sense of touch of the user 220 by causing the controller 234 to vibrate. Also, the feedback unit 214 may simultaneously output only two types of feedback, including feedback of giving stimuli to the sense of sight of the user 220 by the image "excellent!", and feedback of giving stimuli to the sense of touch of the user 220 by causing the controller 234 to vibrate. Alternatively, the feedback unit 214 may simultaneously output only two types of feedback, including feedback of giving stimuli to the sense of hearing of the user 220 by a voice "excellent!", and feedback of giving stimuli to the sense of touch of the user 220 by causing the controller 234 to vibrate.

The action of the user 220 moving the controllers 234 and 235 is a rehabilitation action, and display of a target object for urging the user 220 to do one rehabilitation action that he/she should perform is called a task. Information (task data) representing one task includes the appearance direction of the target object (right 90°, right 45°, front, left 45°, and left 90° with respect to the front direction of the chair), the distance to the target object, the shape (size), the appearance position (the distance and angle from the user), the appearance interval (time interval), the moving speed in falling, rising, or the like, the size, the color, which one of the left and right controllers should be used to acquire, the number of target objects that simultaneously appear, the size of the sensor object, and the like. That is, the task data includes, as information, at least one selected from the group of the moving speed, the number of displayed objects, the size, the display position, and the display interval as the attributes of the target objects 242a and 242b in the virtual space 240. The distance from the user 220 to the fall position of each of the target objects 242a and 242b in the depth direction may continuously be set, or may be set to one of, for example, three stages. For example, a change can be made such that the target object falls quite near the user 220 or falls to a position that the user 220 cannot reach unless largely inclining the body forward. This can control an exercising load to be given to the user, and a load to a spatial cognitive ability or a spatial comprehension ability.

The evaluation updater 215 evaluates the rehabilitation action of the user in accordance with the amount and quality of the task achieved by the user 220 and adds a point. Here, the quality of the achieved task includes "well done" or "excellent", that is, how close the avatar object could be brought to the target object. The evaluation updater 215 adds different points to achieved tasks (a high point to a far object, and a low point to a close object). The evaluation updater 215 can update a task in accordance with the integrated point. For example, a task (the attribute of a target object) may be updated using a task achievement ratio (the number of achieved targets/the number of tasks). The evaluation updater 215 compares the rehabilitation action detected by the action detector 211 and a target position represented by the target object displayed by the display controller 212, and evaluates the rehabilitation capability of the user 220. More specifically, it is decided, by comparing the positions in the three-dimensional virtual space, whether the target objects 242a and 242b and the avatar objects 241a and 241b that move in correspondence with the rehabilitation action detected by the action detector 211 overlap. If these overlap, it is evaluated that one rehabilitation action is cleared, and a point is added. The display controller 212 can make the target objects 242a and 242b appear at different positions (for example, positions of three stages) in the depth direction. The evaluation updater 215 gives different points (a high point to a far object, and a low point to a close object).

The evaluation updater 215 updates a target task in accordance with the integrated point. For example, a target task may be updated using a task achievement ratio (the number of achieved targets/the number of tasks).

The task set database 216 stores a set of a plurality of tasks. A task represents one rehabilitation action that the user should perform. More specifically, as information representing one task, the position of a target object that appears, its speed and size, and the size of an avatar object at that time, and the like are stored. The task set database 216 stores a task set that decides the order of providing the plurality of tasks to the user. For example, task sets may be stored as templates for each hospital, or a history of executed task sets may be stored for each user. The rehabilitation support apparatus 210 may be configured to be communicable with another rehabilitation support apparatus via the Internet. In this case, one task set may be executed by the same user in a plurality of places, or various templates may be shared by a plurality of users in remote sites.

The setter 217 can set such that generation of the left target object 242a and generation of the right target object 242b are alternately performed. The setter 217 sends an instruction to the display controller 213 in accordance with the setting.

The setter 217 can set the generation position of the left target object 242a in the depth direction in the virtual space 240 and the generation position of the right target object 242b in the depth direction in the virtual space 240.

Both the generation timing of the target object and the moving speed of the target object in the vertical direction are controlled, thereby controlling such that the rehabilitation action of the user is always performed alternately on the left and right sides. That is, in the simplest method, the left and right target objects are never generated simultaneously, and instead, after reaching to the right target object is sensed, the left target object is generated. However, the present invention is not limited to this, and various control methods are possible. That is, while simultaneously generating a plurality of target objects, the moving speed is changed such that it is reduced in the order of right → left → right → left, thereby urging the user to do the motion alternately on the left and right sides. For example, after reaching to the right object is sensed, the left object and the right object are simultaneously generated or the right object is generated first. Then, the speed of the right object may be reduced to cause it to take an action of overtaking the left object. A heavy load can thus be applied to the brain. The requester or controller controls such that the rehabilitation action of the user is performed alternately on the left and right sides.

On the other hand, when reaching to the right target object should be done on the lower side and reaching to the left target object should be done relatively on the upper side in a case where, for example, the user has paralysis only on the right side or the left upper body should be actively rehabilitated, after the right target object is generated at a low speed, the left target object may be generated at a high speed at an interval. That is, at least one selected from a group of the generation timing and the speed of the target object is controlled in accordance with the vertical-direction position of the body to be rehabilitated (in accordance with the position of the target object to be reached). In rehabilitation of a knee or the lower half, the generation timing or the speed of the target object is controlled such that reaching is done at a lower position. To increase the cognitive load, the target objects are generated in the order of left → right → right. When only the first target object is moved slowly, and the second and third target objects are moved at a high speed, the user can consequently reach the target objects in the order of right → left → right.

Thus, when rewiring is done in the brain, the symptom of a disease that is difficult to treat in the modern medicine such as chronic pain or restless legs syndrome (an abnormality in basal ganglia) can be improved.

The setter 217 sets at least one selected from a group of the moving speed, the number of displayed objects, the shape, the color, the size, the display position (coordinate information or the like), and the display interval as the attributes of the target objects 242a and 242b in the virtual space 240. The setter 217 may set a delay time from the timing of notifying the generation of the target objects 242a and 242b to the timing of generating the target objects 242a and 242b, thereby controlling a cognitive load given to the user 220. That is, the user needs to continuously memorize and hold an action that he/she should perform during the time after he/she knows, by the radar screen image 250 or the like, a position in the virtual space where the target object is generated (a position representing in which direction the head mounted display should be directed to display the target object) until actual generation of the target object. The "memorization time" is a cognitive load for the user. Also, the setter 217 may control the cognitive load by changing the time not "until the timing of generating the target object 152" but "until the target object 152 approaches the range the user 220 can reach". The setter 217 may give a cognitive load to the user 220 by displaying the background image 243 other than the target objects 242a and 242b on the head mounted display 233. Note that when changing the cognitive load, it is preferable to notify the user in advance that the cognitive load is to be increased or decreased. As for the notification method, the notification may be done by visually using characters or a symbol, by a voice, or by touching a part of the body, for example, by tapping a shoulder, an elbow, an arm, or a foot.

The setter 217 controls the difficulty of the body action in accordance with the motion of a joint different from the body part the user moves to make the body action, that is, the degree of the articular linkage. The setter 217 serving as a controller controls, as point estimation parameters, the size of the target object, the distance from the user, the angle, the speed, the presence/absence of background, the presence/absence of music, and the size of a visual recognition auxiliary image. For example, an articular linkage sometimes largely changes depending on the presence/absence of music. The magnitude of the articular linkage that has occurred is changed even by the type of music. For example, as compared to bird song (this has large influence for a person who is poor in cognition), repeating rhythmic music increases the amount of work of the brain to perform background processing of information, that is, the cognitive load and, therefore, a large articular linkage occurs. The complexity of background information is also largely associated with the amount of work of the brain to perform background processing of information, that is, the cognitive load. For example, if some background or landscape is present in a three-dimensional space, the cognitive load becomes large comparing to the empty space. If a moving animal or object further exists there and the moving animal or object appears in a wider range of the screen, the cognitive load becomes large, and an articular linkage appears more conspicuously. That is, basically, the higher the accuracy of point estimation should be or the larger the load of background processing of sound or background information by the brain is, the smaller the spare capacity of the brain usable for point estimation is. It is therefore known that an articular linkage strongly appears.

Fig. 3A is a view showing a screen (operation panel) 300 to be operated by an operator. The setter 217 displays the operation panel 300. In this example embodiment, point estimation parameters (the distance to the target object, the height of the target object, the direction, the size of the target object, the size of the visual recognition auxiliary image, the moving speed, sensitivity = the size of the sensor object, the generation interval, the type or presence/absence of background information, and the type or presence/absence of a sound effect or BGM) are set by the intuitive operation panel 300, thereby creating a task for supporting rehabilitation. This can be done by one of a manual mode (a method of setting point estimation parameters of each task and performing an operation on a task basis), a template mode (a method of setting point estimation parameters for a plurality of task sets in advance), and an auto mode in which a device is caused to automatically generate a task, or a combination thereof. Note that it is also possible to, on the operation panel, confirm the basic information of the user and various kinds of cognitive and motor functions evaluation indices and examination results, create a template, and set and instruct the auto mode.

The display that displays the operation panel 300 can be any device, and may be an external display connected to the rehabilitation support apparatus 210 or a display incorporated in the rehabilitation support apparatus 210. The operation panel 300 includes a user visual field display region 301, various parameter setting regions 321 to 324, a score display region 303, a re-center button 305, and a BGM control button 307. For a descriptive convenience, Fig. 3A includes a region 306 showing the actual state of the user 220. However, the operation panel 300 need not include the region 306.

The user visual field region 301 shows an image actually displayed on the head mounted display 233. A reference direction in the virtual space is displayed in the user visual field region 301. As described with reference to Fig. 2A, the radar screen image 250 is displayed at the center (for example, within the viewing angle range of -50° to 50°) of the user visual field region 301. The radar screen image 250 shows the relative direction of the positions of the target objects 242a and 242b that appear next with respect to the reference direction in the virtual space. In this example, the coloring position in the block image 252 represents that the target object 242 appears at the farthest position on the left side with respect to the reference direction in the virtual space. Based on the position of the fan-shaped image 253 and the direction of the head image 251, it can be seen that the user already faces left.

The various parameter setting regions 321 to 324 are screens configured to set a plurality of parameters for defining a task. The setter 217 can accept inputs to the various parameter setting regions 321 to 324 from an input device (not shown). The input device may be a mouse, a ten-key pad, or a keyboard, or may be various kinds of controllers, a joystick for game, or a touch panel, and can use any technical component.

The various parameter setting regions 321 to 324 include an input region 320 that decides the sizes of left and right target objects, the input region 321 that decides the size of the visual recognition auxiliary image, the input region 322 that decides the size of the sensor region of each of the avatar objects 241a and 241b, the input region 323 that decides the moving speed of the target object, and the input region 324 that decides the position of a target object that appears next. The operation panel 300 also includes a check box 325 that sets whether to accept an operation of a target object appearance position by various kinds of parameter external input devices such as a hot key and a ten-key pad.

The input regions 320 and 321 can set, on each of the right and left sides, the radius (visual recognition size) of a visual recognition object that makes the target object position easy for the user to see, and the radius (evaluation size) of a target object that reacts with the avatar objects 241a and 241b. That is, in the example shown in Fig. 3A, the user can see a circle with a radius of 20 cm. Actually, the task is completed only when he/she touches a ball with a radius of 10 cm located at the center of the circle. If the visual recognition size is small, it is difficult for the user to find the target object. If the visual recognition size is large, the user can easily find the target object. If the evaluation size is large, the allowable amount of the deviation of the avatar objects 241a and 241b is large. If the evaluation size is small, the allowable amount of the deviation of the avatar objects 241a and 241b is small, and a rehabilitation action can be evaluated more severely. The visual recognition sizes and the evaluation sizes may be made to match. With these settings, the accuracy of feed forward, that is, the accuracy of information processing performed by the brain of the user is quantitatively changed. It is possible to control a treatment approach to the bodily functions (the upper limb function, the walking function, the trunk function, and the balance function), the cognitive functions (including the spatial cognition and attention functions), and the sensory functions (including the inner ear, the vestibular system, the tactile sensation, the temperaesthesia and pain sensation, the position sensation, and the depth sensation) and the degree of visualization of an articular linkage.

In the input region 322, the left and right sensor sizes of the avatar objects 241a and 241b (the size of the sensor object) can be set. If the sensor size is large, a task is achieved even if the position of a hand largely deviates from the target object. Hence, the difficulty of the rehabilitation action is low. Conversely, if the sensor size is small, it is necessary to correctly move the hand to the center region (evaluation size) of the target object. Hence, the difficulty of the rehabilitation action is high. In the example shown in Fig. 3A, the sensor sizes are 2 cm on the left and right sides. These elements also quantitatively change the accuracy of feed forward, that is, the accuracy of information processing of the brain and makes it possible to control a treatment approach to the bodily functions (the upper limb function, the walking function, the trunk function, and the balance function), the cognitive functions (including the spatial cognition and attention functions), and the sensory functions (including the inner ear, the vestibular system, the tactile sensation, the temperaesthesia and pain sensation, the position sensation, and the depth sensation) and the degree of visualization of an articular linkage.

In the input region 323, the speed of the target objects 242a and 242b moving in the virtual space can be defined on the left and right sides. In this example, the speed is set to 45 cm/s.

The input region 324 is an image used to input the position of the next task (the distance to the task and the angle), and has the shape of the enlarged radar screen image 250. Since the check box 325 has a check mark, an operation by the input device can be performed. If an operation of clicking or tapping one of a plurality of blocks in the input region 324 is performed, or an operation corresponding to this is performed by the input device, the target objects 242a and 242b are generated at a position in the virtual space corresponding to the position of the designated block.

That is, in the example shown in Fig. 3A, the task for the user 220 is to bring an avatar object (controller) including a sensor portion with a size of 2 cm into contact with a target object (ball) having a radius of 10 cm that falls at a speed of 45 cm/s in a far place on the left side in a good timing in the virtual space. Fig. 3A shows a state in which the target object 242a appears in the user visual field region 301. In this state, when the user 220 stretches the left arm, as shown in the region 306, the avatar object 241a appears in the user visual field region 301. A visual recognition object 312 that raises the visibility of the target object 242a is displayed around the target object 242a. The visual recognition size set in the input region 321 is the radius of the visual recognition object 312 with a doughnut shape. Note that the target object and the avatar object can correspond to either left or right, and the user may be urged to do an operation of moving the left side of the body to the same side, that is, the left side in the virtual space or may be urged to do an operation of moving it to the right side.

At the point of time when the avatar object 241a contacts the visual recognition object 312, the target object 242a does not disappear, and perfect achievement of the task is not obtained (the achievement is imperfect, a predetermined point is added, and good evaluation is done). Perfect achievement of the task (perfect evaluation) is obtained only when the avatar objects 241a and 241b contact the target objects 242a and 242b.

On the other hand, the total number of tasks at each position, and a count point representing how many times a task has been achieved are shown in the score display region 303. The point may be expressed as a fraction or a percentage, or a combination thereof. After a series of rehabilitation actions decided by one task set, the evaluator 214 derives a rehabilitation evaluation point using values in a score list 332.

The re-center button 305 is a button that accepts, from the operator, a reconstruction instruction for reconstructing the virtual space in accordance with the position of the user 220. If the re-center button 305 is operated, the display controller 212 sets the position of the head mounted display 233 at the instant to the origin, and reconstructs the virtual space having, as the reference direction, the direction of the head mounted display 233 at that instant. By operating the BGM control button 307, background sound during task execution can be turned on/off. When the background sound is eliminated, the load of the task can be reduced, and the user can be made to concentrate to the motion of the body. In general, if background sound is turned off for a user with an articular linkage, the articular linkage during the task tends to be reduced. Reversely, it is also possible to make an articular linkage appear by turning on background sound for a user who has articular linkage.

The display controller 213 may display an information bar 310 on the display screen 240 of the head mounted display 233, in addition to the radar screen image 250. The information bar 310 displays a player name 311, a task achievement level 312, and an elapsed time 313 from the start of play. The information bar 310 is parallel to the horizon 244 in the virtual space. If the head position or body axis of the patient is distorted, the parallel relationship between the radar screen image 250 following the tilt of the head mounted display 233 and the information bar 310 breaks. For this reason, the patient can correct inner ear information by vision assistance, and the therapist readily notices the distortion of the body axis of the patient. Fig. 3A exemplifies an image when the head position tilts by 20° to the right side. The user interface of the head mounted display 233 is designed such that not only elements for allowing the patient to deeply engage in rehabilitation and but also medical utility are provided.

Fig. 3B is a view showing another example of the operation panel 300. As indicated by an articular linkage message 308, the presence/absence of an articular linkage and the size thereof may be detected by a camera or the like and notified to the operator as characters, an image, or a color change. As described above, a heat map may be superimposed on a user image 306, and an image region where the possibility of an articular linkage is high may be displayed in red.

The procedure of controlling the accuracy of point estimation for an articular linkage is as follows. (1) A rehabilitation action is performed by default settings. (2) An articular linkage is too large (the angle or distance of bowlegs, the twist of a shoulder, the twist of the hip, the twist of the head, or the like exceeds a threshold). (3) The requested difficulty of point estimation is lowered (the point estimation parameters are changed). (4) The articular linkage moderately decreases. (5) The rehabilitation action is repeated alternately on the left and right sides. (6) The articular linkage is eliminated. (7) The requested accuracy of point estimation is raised (the point estimation parameters are changed). (8) The articular linkage obtains a moderate magnitude. (9) The process returns to (5).

Note that in (5), the articular linkage may be suppressed by call-out or physical contact to the body of the user. Also, in (7), a load may be applied to the user by call-out to obtain the same effect as in raising the requested accuracy of point estimation. It has become known that the articular linkage of the user can quickly be eliminated by touching the body of the user.

As described above, the direct visual field of the user is blocked, point estimation is performed, a strong motion instruction is issued via a target object, the deep muscle contracts, and alignment of the whole body is obtained. At this time, an error in the cranial nerve, called an articular linkage, is found, and the rehabilitation action is repeated while controlling the requested accuracy (various kinds of parameters) of point estimation until the articular linkage is reduced.

If a plurality of articular linkages appear, it is judged which articular linkage should mainly be corrected. For example, a knee (hip joint) that has largely moved is corrected first, and the process advances to the next joint (for example, the sole of a foot faces inside due to an involuntary movement of an ankle joint).

When the rehabilitation action is performed aiming at recovering the articular linkage, the cognitive and exercise ability of the user can be improved (the disorder in the brain can be corrected) very effectively. The articular linkage is the guideline for the treatment. A stimulus (point estimation request) is imparted to the brain, and the treatment plan is judged based on the reaction of the articular linkage. An effective treatment can be made by actively contacting the body part where an articular linkage appear.

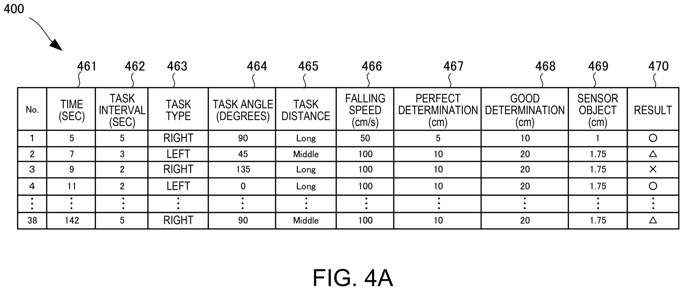

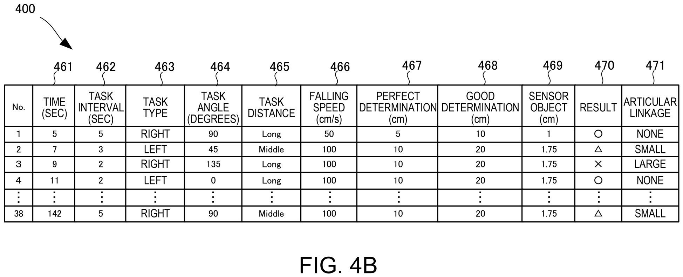

Fig. 4A is a view showing a task table 400 stored in the task set database 216. In the task table 400, a time (task generation timing) 461, a task interval 462 from the end of an immediately preceding task, a task type 463, a task angle 464, and a task distance (intensity) 465, and a task distance (intensity) 465 are stored in linkage with a task ID. Also, in the task table 400 a target object speed 466, a perfect determination (excellent evaluation) reference size 467, a good determination (well done evaluation) reference size 468, a sensor object size 469, a task achievement result, and the like are stored in linkage with a task ID. In addition to these, a delay time (predetermined time) from task generation notification to task generation may be set for each task. Fig. 4B is a view showing another example of the task table 400 stored in the task set database 216. As shown in Fig. 4B, data 471 associated with an articular linkage may be accumulated.

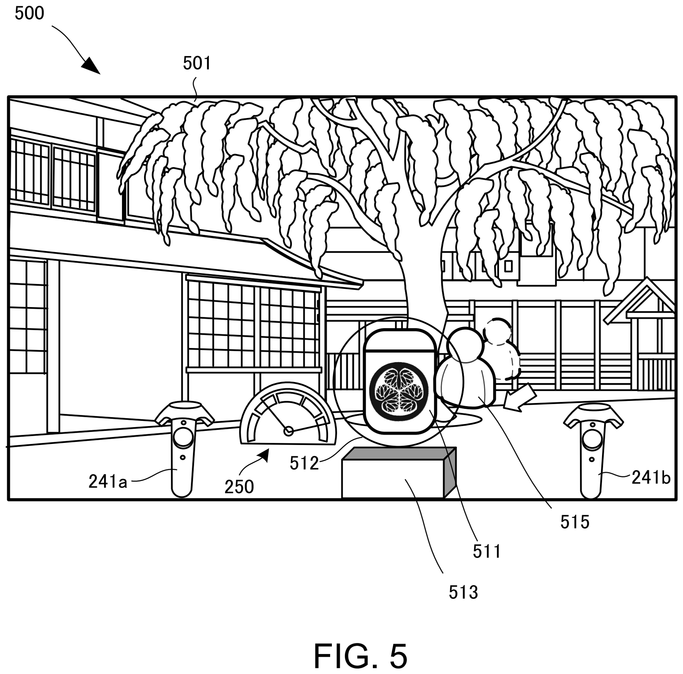

Figs. 5 to 7 are views showing examples of display on the head mounted display 233 according to this example embodiment. Both addition of such background information and addition of a background sound as one execution method impart a load to information processing of the brain and causes a conspicuous articular linkage. In Fig. 5, an image showing an inro 511 as a target object is displayed on a background image 501 expressing a street in the Edo period. In addition, under the inro 511, a sen-ryo-bako 513 is displayed as an item to be protected by the user, and a ninja 515 gradually comes close from the far side. The speed of the ninja 515 is the speed set in the input region 323 of the operation panel 300 (the speed here has the same meaning as a time limit). A circle 512 serving as a visual recognition assisting image is displayed on the inro 511. If the user touches the inro 511 by the sensor object (the center of the tip of the avatar object 241a or 241b) before the ninja 515 reaches the sen-ryo-bako 513, the task is achieved. Two types of circles, that is, red and blue circles are prepared as the circle 512. The task for the inro 511 surrounded by the red circle 512 is to operate the red avatar object 241b on the right side, which corresponds to the controller 235 held in the right hand, and bring it into contact with the inro 511. On the other hand, the task for the inro 511 surrounded by the blue circle 512 is to operate the red avatar object 241a on the left side, which corresponds to the controller 234 held in the left hand, and bring it into contact with the inro 511.

The inro 511 is displayed at a position (depth and angle) set in the input region 324 of the operation panel 300. The position of the inro 511 does not change until the user makes the avatar object 241a or 241b touch it in the virtual space. That is, the inro 511 is a target object fixed in the space (also called a horizontal task because the user is required to horizontally stretch the body). Such a fixed target object is very effective as rehabilitation for a disease such as cerebellar ataxia, diplopia, or inner ear dysfunction. That is, an image of a limited body motion can be imprinted, by feed forward, in the brain of a patient who has forgotten how to move his/her body. If the distance of the inro 511 in the depth direction is increased, the motion intensity can be changed. Also, if multi-channel biofeedback is combined, the exercise ability, the bodily functions, the cognitive functions, and the sensory functions greatly improve. Furthermore, according to such a horizontal task, chronic pains can be improved by promoting reconstruction of cerebral cortex. Alternatively, it is possible to recover cognitive impairment called chemobrain, a sensory dysfunction in which the position sense of a cancer patient using an anticancer drug lowers due to neuropathy, or an aftereffect symptom caused by infection of COVID-19. The place where the target object appears may be notified in advance to give a hint and reduce the cognitive load. Not language informing but touch informing by touching the body, language informing repeated a plurality of times, and a combination thereof are also effective to reduce the cognitive load. As for the method of language informing, the cognitive load may be reduced by giving a simple short instruction close to the imperative form. Alternatively, a more complex instruction may be given in a questioning format, for example, using "blue? (take by the right hand)". Language informing may be given in a form including a cognitive task such as calculation, for example, "take by the right hand if you hear a number divisible by 2". Note that not only the horizontal position and depth where the inro 511 is generated but also a height may be set.

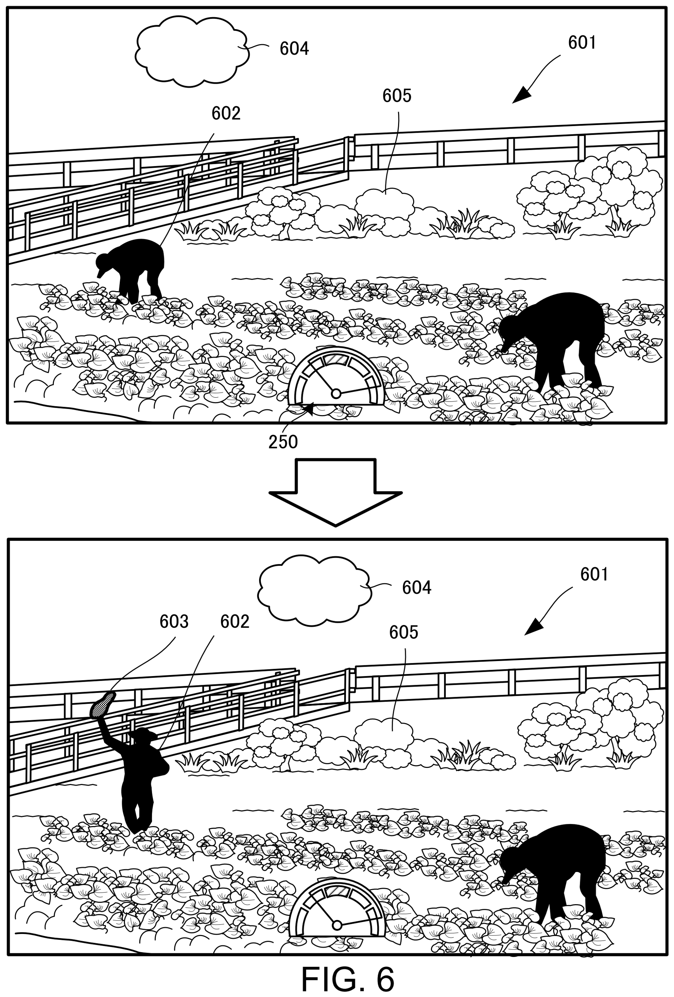

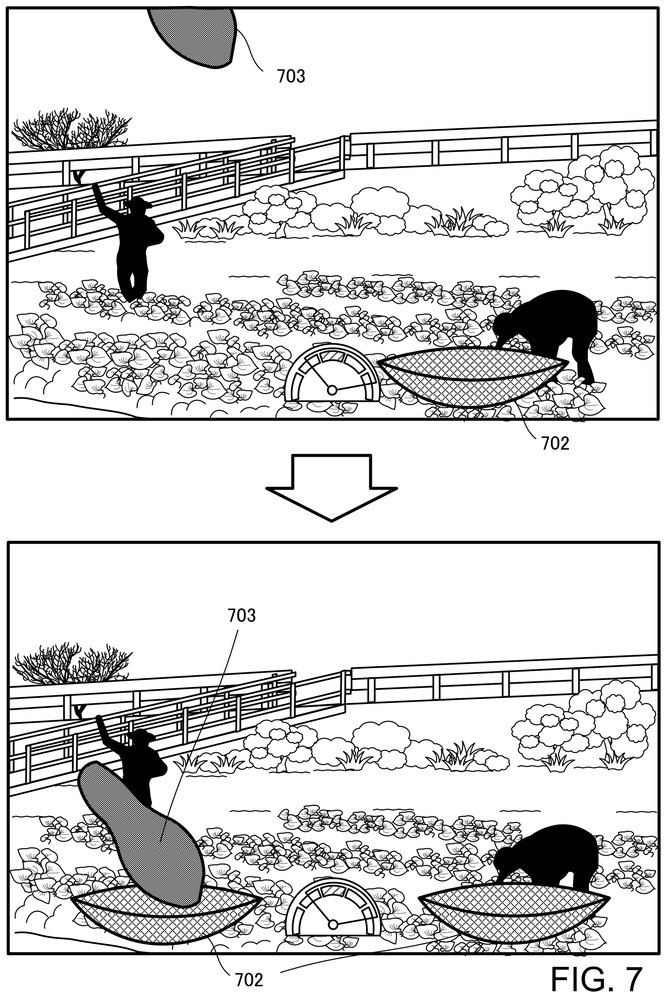

Fig. 6 is a view showing an example (vertical task or falling task) of a screen for performing a task in which a target object moves vertically. In Fig. 6, on a background image 601 representing a field, an image of a person representing a farmer is displayed as a trigger object 602 serving as a trigger of target object appearance. That is, the display controller 213 displays the trigger object 602 as a notification image used to notify the user of generation of a target object 603. When a predetermined time elapses after the trigger object 602 throws up the target object 603 in the shape of a potato, a target object 703 having the shape of a large potato appears from the upper side of the screen, as shown in Fig. 7. When the falling target object 703 is received by moving an avatar object 702 having the shape of a basket, the task is achieved. The left and right avatar objects 702 move on the screen in synchronism with the motions of the controllers 234 and 235.

The setter 217 sets the delay time from the timing at which the trigger object 602 throws up the target object 603 and notifies the generation of the target object 603 until generation of the target object 703, and the cognitive load given to the user can thus be adjusted. The longer the delay time is, the longer the period to hold the memory is and the more the load of information processing of the brain increases. Note that in synchronism with the motion of the trigger object 602, generation of the target object may be notified at the same timing using the radar chart type notification image 250, or a notification by a voice may be combined.

In this way, the setter 217 can give a cognitive load to the user not only by a task of a background including only a horizontal line as shown in Fig. 2 but by a task of a background including a large quantity of information as shown in Figs. 5 and 6. That is, it is made difficult to memorize that the target object 603 has appeared, and the position to which the target object 703 is expected to fall, thereby giving a load closer to a cognitive load necessary in a real life to the user of rehabilitation.

In particular, the setter 217 changes the mode of the task and changes at least a part of the background image 301 along with time, thereby giving a cognitive load for processing the background image in the brain to the user 220. In the example shown in Fig. 6, for example, in the background image 601, a cloud 604 may be moved, plants 605 may be shaken, or an animal (not shown) irrelevant to the target object may be made to appear. This can impede concentration to the target object 603 and make it more difficult for the user 220 to memorize the position to which the target object 603 is expected to fall. More technically, it can be said that information irrelevant to the task is displayed on the background image to prepare an environment in which it is difficult to concentrate to the target object and intentionally cause an attention disorder (more specifically, a selective attention disorder, a divided attention disorder, a conversion attention disorder, or a sustained attention disorder), thereby making memorization difficult and controlling the cognitive load.

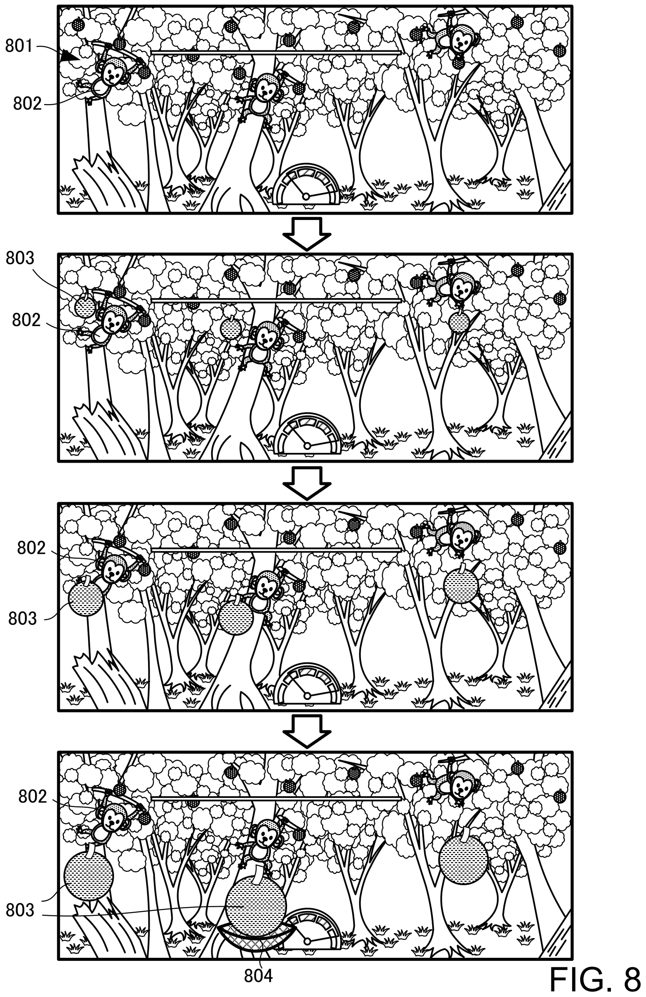

Fig. 8 is a view showing another example (vertical task or falling task) of display on the display screen 240 according to this example embodiment. In Fig. 8, in a background image 801 like woods, a trigger object 802 representing a monkey and a target object 803 representing an apple are displayed. When the trigger object 802 representing a monkey drops the target object 803 representing an apple from a tree, and the target object 803 approaching the user is received by moving an avatar object 804 representing a basket, the task is achieved. In this case as well, the setter 217 starts dropping the target object 803 after the elapse of a predetermined time from the timing at which the trigger object 802 shakes the tree and notifies the generation of the target object 803, thereby giving a cognitive load to the user 220 while causing an attention disorder.

Also, the setter 217 causes at least two to five target objects 803 to simultaneously exist in the three-dimensional virtual space and displays these on the display screen 240, thereby giving a cognitively very strong load to the user 220. In other words, the setter 217 generates the at least two target objects 803 at different positions in the left-and-right direction in the three-dimensional virtual space.

In particular, if the at least two target objects 803 are generated at a plurality of positions in a direction (the left-and-right direction in Fig. 8) different from the moving direction (the falling direction in Fig. 5) of the target object 803, a larger cognitive load can be given. That is, since the user 220 needs to move the controllers 234 and 235 in consideration of the movement in the vertical direction, the difference between the generation positions in the left-and-right direction, and the difference in the falling position in the depth direction, the spatial cognitive ability is also tested. As described above, the type, number, size, spatial spread, position, amount, and the like of information included in a notification image including a trigger object or a notification sound are adjusted in addition to the change of the predetermined time of the task, thereby quantitatively adjusting and controlling the complexity of information to be memorized and held, that is, a cognitive load that should be subjected to information processing by the brain of the user.

The evaluation updater 215 evaluates the cognitive ability of the user using information such as whether the avatar object has reached, in a good timing, a three-dimensional target position represented by the target object, the time interval from target object generation notification to generation and the number of target objects, and the degree of the load that causes the attention disorder on the background image.

The requested accuracy of point estimation changes depending on which one of the various modes (display screens) shown in Figs. 2A, and 5 to 8 should be executed by the user. For example, as compared to a case where there is no background, as shown in Fig. 2A, the requested accuracy of point estimation increases in the order of Fig. 5 → Fig. 8 → Fig. 6. Also, as described above, the requested accuracy of point estimation changes depending on the background sound, and this affects articular linkages.

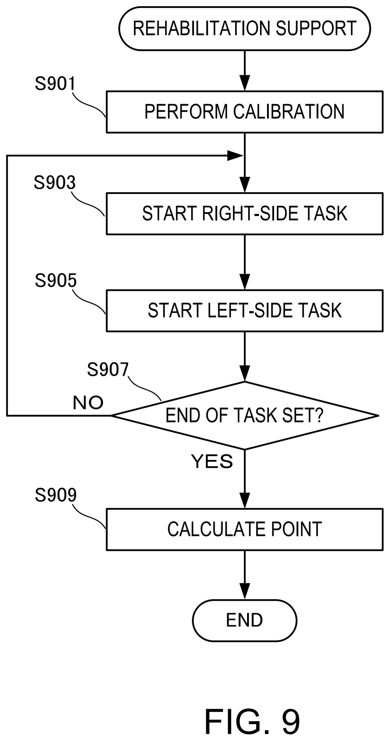

Fig. 9 is a flowchart showing the procedure of processing of the rehabilitation support apparatus 210. In step S901, as calibration processing, the target of a rehabilitation action is initialized in accordance with the user. More specifically, each user is first made to do a work for acquiring an action enable range as calibration. The range is set to the initial value, and the target is initialized in accordance with the user. The initial value may be set in accordance with the action enable range of the user, or the therapist who provides the treatment may decide the initial value in consideration of the user's body cognitive functions or a target treatment effect.

Next, in step S903, a right-side task (that is, display of the target object by the display controller 212 and evaluation of achievement by an avatar object) is started.

In step S905, a left-side task (that is, display of the target object by the display controller 212 and evaluation of achievement by an avatar object) is started.

In step S909, the task achievement level is acquired, and a point is added. Note that after the occurrence of the task on one side, the task on the opposite side may be generated without waiting evaluation of the achievement, and these may be processed parallelly or with a time lag. The first task can started either on the left or right side, and the last task can also be performed either on the left or right side. That is, the procedure described here is merely an example, and the present invention is not limited to this.

### <Data>

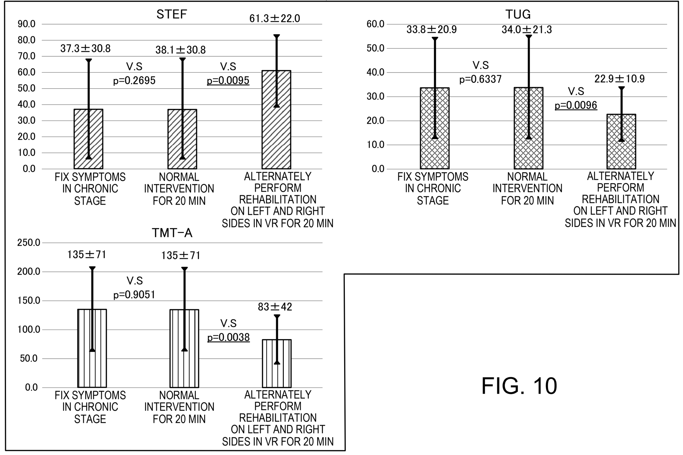

Fig. 10 is a view showing a result obtained using the rehabilitation support system 200 according to this example embodiment.

Cases were n = 13. As the composition, the average age was 60 ± 21, the number of male patients was 7 (53.9%), the number of cerebrovascular disease patients was 5 (38.4%), the number of orthopedic disease patients was 4 (30.8%), and the number of neurodegenerative diseases patients was 4 (30.8%). All patients had gone through normal rehabilitation for a half year or more, and it was said that it is difficult to obtain more improvement for these people. Even when rehabilitation for intensively training the affected side for first 20 min was conducted for the patient group, STEF, TUG, and TMT-A were not improved with statistical significance (the graph of normal intervention for 20 min in Fig. 10). Here, STEF (Simple Test for Evaluating Hand Function) is the upper limb function test. TUG (Timed Up and Go test) is a comprehensive inspection index for the walking function, the trunk function, and the balance function. TMT-A (Trail-Making Test-A) is a kind of cognitive function inspection. STEF is an index indicating a good score by a large numerical value, and TUG and TMT-A are each an index indicating a good score by a small numerical value. All data are indicated by average ± standard deviation. For statistical analysis, paired-t test that is an average comparison test considering pairs was used, and a standard with a statistical significant difference was p < 0.05.

On the other hand, when the patients were caused to perform rehabilitation alternately on the left and right sides while being conscious of the depth, using the system 200 according to this example embodiment regardless of whether it was the affected side or the unaffected side, all the STEF, TUG and TMT-A were improved.

That is, as in this example embodiment, it was found that, when a patient performs training alternately on the left and right sides while being conscious of the depth, not only the upper limb function but also the trunk function and the balance function improve, and the waling function (bodily function) improves in turn, and furthermore, the spatial cognitive ability and the attention function improve. Note that it was guessed that, for example, when improving STEF, TUG, an TMT-A, improvement of the inner ear, the vestibular system, the tactile sensation, the temperaesthesia and pain sensation, the position sensation, and the depth sensation has influence. It was thus proved that to stimulate brain plasticity at maximum and maximize the efficiency of motion learning, cognitive learning, and sensation learning of the brain, it is effective that the patient performs rehabilitation alternately on the left and right sides while being conscious of the depth. Note that it was found, from other experimental results and the like, that when the treatment according to the contents of the present application is performed alternately on the left and right sides, improvement can quickly be obtained as compared to an otherwise case. It can also be considered that the symmetrical distribution of motor areas shown in non-patent literature 1 or Fig. 2B is a finding supporting the fact.

### [Other Example Embodiments]

While the invention has been particularly shown and described with reference to example embodiments thereof, the invention is not limited to these example embodiments. It will be understood by those of ordinary skill in the art that various changes in form and details may be made therein without departing from the spirit and scope of the present invention as defined by the claims. A system or apparatus including any combination of the individual features included in the respective example embodiments may be incorporated in the scope of the present invention.

The present invention is applicable to a system including a plurality of devices or a single apparatus. The present invention is also applicable even when an information processing program for implementing the functions of example embodiments is supplied to the system or apparatus directly or from a remote site. Hence, the present invention also incorporates the program installed in a computer to implement the functions of the present invention by the computer, a medium storing the program, and a WWW (World Wide Web) server that causes a user to download the program. Especially, the present invention incorporates at least a non-transitory computer readable medium storing a program that causes a computer to execute processing steps included in the above-described example embodiments.

This application is based upon and claims the benefit of priority from Japanese patent application No. 2023-97169, filed on June 13, 2023, the disclosure of which is incorporated herein in its entirety by reference.

## Claims

1. An information processing system comprising:
a blocker that makes whole or a part of a body of a user invisible;
a requester that requests the user to perform a body action that makes a part of the body of the user or an object operated by the user overlap a target object; and
a controller that controls, under an environment which is generated by said blocker and said requester and in which an abnormal action of a joint is visualized, difficulty of the body action in accordance with a phenomenon called an articular linkage in which a joint that is unnecessary for a motion requested by said requester unintentionally moves.

2. The information processing system according to claim 1, further comprising a feedback unit that feeds back achievement of the body action requested by said requester to the user.

3. The information processing system according to claim 2, wherein said feedback unit performs feedback of stimulating not less than two sensations of five senses of the user every time the body action requested by said requester is achieved.

4. The information processing system according to claim 1, further comprising an articular linkage evaluator that evaluates the articular linkage.

5. The information processing system according to claim 1, wherein said requester or said controller controls such that the body action of the user is performed alternately on left and right sides.

6. The information processing system according to claim 1, wherein said controller controls the difficulty of the body action by changing at least one selected from a group of a size of the target object, a distance from the user, an angle, a speed, presence/absence of background, presence/absence of music, and a size of a visual recognition auxiliary image.

7. The information processing system according to claim 1, wherein
said blocker is a head mounted display that displays a virtual space, and
said requester generates, in the virtual space, the target object and an avatar object representing the part of the body of the user or the object operated by the user and displays the target object and the avatar object on said head mounted display.

8. The information processing system according to claim 1, wherein
said blocker is a projection mapping system that displays a part of background on the whole or the part of the body of the user or a contact lens type display that displays a virtual space, and
said requester displays, using said projection mapping system, the target object and an avatar object representing the part of the body of the user or the object operated by the user on the background, or generates, in the virtual space, the target object and the avatar object representing the part of the body of the user or the object operated by the user and displays the target object and the avatar object on said contact lens type display.

9. An information processing method by an information processing system including a blocker, a requester, and a controller, comprising:
making, by the blocker, whole or a part of a body of a user invisible;
requesting, by the requester, the user to perform a body action that makes a part of the body of the user or an object operated by the user overlap a target object; and
controlling, by the controller, under an environment which is generated in the making and the requesting and in which an abnormal action of a joint is visualized, difficulty of the body action in accordance with a phenomenon called an articular linkage in which a joint that is unnecessary for a motion requested by the requester unintentionally moves.

10. An information processing program for causing a computer to execute a method, comprising:
making whole or a part of a body of a user invisible;
requesting the user to perform a body action that makes a part of the body of the user or an object operated by the user overlap a target object; and
controlling, under an environment which is generated in the making and the requesting and in which an abnormal action of a joint is visualized, difficulty of the body action in accordance with a phenomenon called an articular linkage in which a joint that is unnecessary for a motion requested in the requesting unintentionally moves.
